# EUROPÄISCHE PATENTSCHRIFT

(11) **EP 1 191 017 B1**
(45) Veröffentlichungstag und Bekanntmachung des Hinweises auf die Patenterteilung: **20.12.2006**
(21) Anmeldenummer: 01119006.3
(22) Anmeldetag: 07.08.2001
(51) Int. Cl.: C07C 249/04, C07C 251/44

(54) **Verfahren zur Herstellung von Oximen**
Process for the production of oximes
Procédé de préparation d'oximes

(30) Priorität: 26.09.2000 DE 10047435
(43) Veröffentlichungstag der Anmeldung: 27.03.2002
(73) Patentinhaber: Degussa AG, 40474 Düsseldorf (DE)
(72) Erfinder: Schiffer, Thomas, Dr., 45721 Haltern (DE); Thiele, Georg Friedrich, Dr., 63450 Hanau (DE); Hasenzahl, Steffen, Dr., 63477 Maintal (DE)

(56) Entgegenhaltungen:
- EP-A- 0 267 362
- DE-A- 4 240 691
- DE-A- 4 240 698
- PRASAD R ET AL: "AMMOXIMATION OF CYCLOHEXANONE OVER AL2O3 SUPPORTED TITANIUM SILICATES" JOURNAL OF CHEMICAL TECHNOLOGY AND BIOTECHNOLOGY. (INTERNATIONAL JOURNAL OF BIOTECHNICAL AND CHEMICAL PROCESSES), ELSEVIER APPLIED SCIENCE PUBLISHERS. BARKING, GB, Bd. 68, Nr. 3, 1. März 1997 (1997-03-01), Seiten 310-314, XP000700650 ISSN: 0268-2575
- DATABASE WPI Section Ch, Week 198831 Derwent Publications Ltd., London, GB; Class A41, AN 1988-217530 XP002235878 & JP 63 154653 A (MITSUBISHI CHEM IND LTD) , 27. Juni 1988 (1988-06-27)

## Beschreibung

Die Erfindung betrifft ein katalytisches Verfahren zur Herstellung von Oximen. Dabei wird eine Carbonylverbindung, vorzugsweise ein Cycloalkanon mit 7 bis 20 Kohlenstoffatomen, in flüssiger Phase an einem heterogenen Katalysatorsystem, welches aus zwei oder mehr Komponenten besteht, wovon mindestens eine der Komponenten aus mindestens einem porösen, titanhaltigen Festkörper und mindestens eine zweite Komponente aus einem sauren Festkörper besteht, mit Ammoniak und Wasserstoffperoxid umgesetzt (Ammoximation).

In den europäischen Patentanmeldungen EP-A-0 208 311, EP-A-0 267 362 und EP-A-0 299 430 sowie in der amerikanischen Patentschrift US 4 794 198 wird die Darstellung und Aktivierung eines Katalysators auf Basis von Titan, Silizium und Sauerstoff sowie dessen Verwendung zur Synthese von Oximen ausgehend von Aldehyden oder Ketonen wie beispielsweise Cyclohexanon durch Umsetzung mit Wasserstoffperoxid und Ammoniak beschrieben. Die Katalysatoren weisen üblicherweise ein Verhältnis Silizium : Titan größer 30 auf. Ein typischer Vertreter ist der Titansilikalit TS1.

Während die Synthese kleiner aliphatischer und cycloaliphatischer Oxime ausgehend von Ketonen mit bis zu 6 Kohlenstoffatomen wie zum Beispiel Cyclopentanon und Cyclohexanon gute Ergebnisse an zahlreichen, gemäß oben genannten Schriften dargestellten und aktivierten Titansilikaliten als Katalysator liefert, fallen die Resultate bei größeren oder sterisch anspruchsvolleren Carbonylverbindungen wie zum Beispiel Acetophenon und Cyclododecanon deutlich schlechter aus. Insbesondere sind die Reaktionsgeschwindigkeit, der Umsatz der eingesetzten Carbonylverbindung und die Ausbeute bezüglich Wasserstoffperoxid (zur Ammoximation verwendetes H₂O₂ : Gesamtmenge benötigtes H₂O₂·100 %) bei diesen Versuchen unbefriedigend.

Werden bei Cyclohexanon in den Beispielen der europäischen Patentanmeldung EP-A-0 267 362 Umsätze von über 90 % bei einem Peroxidverlust von unter 10 % erzielt (Beispiele 22 und 24), so werden bei vergleichbaren Reaktionsbedingungen mit Acetophenon nur noch Umsätze von 50,8 % bei einem Peroxidverlust von 48,9 % erreicht. Die Umsetzung von Cyclododecanon wird in der genannten Anmeldung beansprucht, jedoch kein konkretes Beispiel zu Umsatz und Peroxidverlust angegeben.
Die deutlich schlechteren Ergebnisse bei großen oder sterisch anspruchsvollen Carbonylverbindungen lassen sich unter anderem darauf zurückführen, dass große Carbonylverbindungen wie Cyclododecanon nicht oder nur langsam durch die Poren des Titansilikalit-Katalysators dringen können. Dadurch kann es zu einer räumlichen Trennung der Teilschritte Hydroxylaminbildung (1) und Oximierung des Ketons (2) (in den Reaktionsgleichungen dargestellt am Beispiel des Cyclododecanons (CDON)), kommen.

Die unproduktive Zersetzung des Hydroxylamins mit vorhandenem Wasserstoffperoxid, formal dargestellt als stöchiometrische Gleichung (3), kann zu einem erheblichen Anteil als Konkurrenzreaktion auftreten, was vor allem die Ausbeute bezogen auf das Peroxid senkt.

(1) NH₃ + H₂O₂ → H₂O + NH₂OH

(2) NH₂OH + CDON → CDON-Oxim + H₂O

(3) 2 NH₂OH + H₂O₂ → 4 H₂O + N₂

Enichem beansprucht in der deutschen Patentanmeldung DE 195 21 011 A1 (entspricht US 5 498 793) ein amorphes Siliziumdioxid als Cokatalysator zur Ammoximation von Acetophenon und Cyclododecanon. Durch Zugabe von amorphem Siliziumdioxid lässt sich danach der Umsatz bei Cyclododecanon nach 8 Stunden Reaktionszeit auf 85,5 % beziehungsweise 85,2 % steigern (DE 195 21 011, Beispiele 5 und 6) im Vergleich zu 76,6 % ohne Cokatalysator. Die Peroxidausbeute steigt gleichzeitig von 65,8 % auf 71,4 % beziehungsweise 72,3 % an.

Das von Enichem beschriebene Verfahren führt zu einer leichten Verbesserung von Umsatz und Peroxidausbeute, doch weist die beschriebene Reaktionsführung mehrere Nachteile auf, die einen technischen Einsatz unwirtschaftlich erscheinen lassen:
■ Die Menge an Katalysator und an Cokatalysator bezogen auf das eingesetzte Keton ist in den Beispielen mit je bis zu 25 Gew.-% bei Versuchen mit Cyclododecanon sehr hoch.
■ Trotz der hohen Katalysatorkonzentration ist die Umsatzrate gering und die Reaktion langsam.
■ Auch nach einer Gesamtreaktionszeit von 8 Stunden liegt die Oxim-Ausbeute noch weit von einem vollständigen Umsatz entfernt (als vollständig kann ein Umsatz von etwa 99 %, nach Möglichkeit über 99,5 % angesehen werden).

Als Umsatzrate ergibt sich über die 8 Stunden Reaktionszeit ein Mittelwert von 7,10 beziehungsweise 7,13 mg Oxim / (g Kat · min) gegenüber 6,38 mg Oxim / (g Kat · min) ohne amorphes Siliziumdioxid als Cokatalysator.

Hohe Umsatzraten, die zu einem vollständigen Umsatz führen, sind für einen technischen Einsatz bei größeren Ringen wie zum Beispiel bei Cyclododecanon sehr wichtig, da sich mit steigendem Molekulargewicht das unumgesetzte Keton vom entsprechenden Oxim nur noch mit großem technischen Aufwand abtrennen lässt.

Es bestand daher die Aufgabe, ein Verfahren aufzufinden, bei dem die Ammoximation mit möglichst vollständigem Umsatz bei gleichzeitig hoher Umsatzrate und guter Peroxidausbeute abläuft. Der Umsatz sollte dabei nach Möglichkeit so hoch sein, dass auf eine Nachreaktion mit einer wässrigen Hydroxylaminlösung verzichtet werden kann.

Überraschend wurde nun gefunden, dass die Aufgabe dadurch gelöst werden kann, dass ein saurer Cokatalysator zusammen mit dem titanhaltigen Katalysator verwendet wird. Insbesondere wurde gefunden, dass sich die Umsatzrate dadurch deutlich verbessern lässt.

Gegenstand der Erfindung ist daher ein Verfahren zur Herstellung von Oximen aus cyclischen Ketonen mit 7 bis 20 Kohlenstoffatomen, Wasserstoffperoxid und Ammoniak in Gegenwart eines Katalysatorsystems, das aus mindestens zwei Komponenten besteht, wobei als Katalysator mindestens eine Komponente aus mindestens einem Kristallinen mikro- oder mesoporösen Feststoff auf Basis von Titan, Silizium und Sauerstoff aufgebaut ist, und als Cokatalysator mindestens eine weitere Komponente aus einem sauren Feststoff, der ein organisches oder anorganisches Trägermaterial enthält, besteht, wobei entweder das Trägermaterial selbst lewis- oder brönstedsaure Eigenschaften besitzt oder entsprechende lewis- oder brönstedsaure funktionelle Gruppen auf das Trägermaterial aufgebracht werden und die Auftragung solcher Gruppen sowohl physikalisch als auch chemisch erfolgen kann, und wobei keine zusätzliche Aktivierung des Katalysators vor der Reaktion erfolgt.

Bevorzugte Katalysatoren auf Basis von Titan, Silizium und Sauerstoff sind Verbindungen mit poröser Struktur, beispielsweise Titansilikalite. Dabei können sowohl mikroporöse als auch mesoporöse Strukturen vorhanden sein. Als nicht limitierende Beispiele für mikroporöse Titansilikalite sind die Typen TS 1 und Ti-beta zu nennen. Nicht limitierende Beispiele für mesoporöse Strukturen sind die Titansilikalite der Typen Ti-MCM41 und Ti-HMS.

Als Cokatalystor eignen sich Festkörper, die an der Oberfläche oder in Poren lewis- und/oder brönstedsaure Zentren aufweisen. Als nicht limitierende Beispiele für anorganische Cokatalysatoren seien saure Aluminiumoxide und saure, aktivierte Alumosilikate wie Bentonit, Montmorrillonit und Kaolinit genannt. Als nicht limitierende Beispiele für Cokatalysatoren, die auf organische Trägermaterialien aufgebaut sind, seien saure und stark saure Ionenaustauscherharze wie Amberlyst 15 oder Nafion NR50 genannt.

Katalysator und Cokatalysator können jeweils als Feststoff sowohl als Pulver als auch als Formkörper verwendet werden. Das Gewichtsverhältnis zwischen Katalysator und Cokatalysator variiert üblicherweise zwischen 0,1 : 1 und 10 : 1.

Werden Katalysator und/oder Cokatalysator als Formkörper eingesetzt, können optional weitere Komponenten als Zuschlagstoffe vorhanden sein, die im Formkörper als Binder fungieren. Als nicht limitierende Beispiele für solche Komponenten seien neutrale und/oder schwach saure Silikate, Alumosilikate und Tonmineralien genannt.
In einer besonders bevorzugten Variante der Erfindung übernimmt ein saurer Feststoff gleichzeitig die Funktionen des Cokatalysators und des Binders für den Titansilikalit-Formkörper.

Selbstverständlich können sowohl der Katalysator als auch der Cokatalysator aus Mischungen von zwei oder mehr Komponenten bestehen.

Das Verfahren der vorliegenden Erfindung erlaubt die Ammoximation von großen Carbonylverbindungen und bevorzugt von großen cyclischen Ketonen, insbesondere von Ringen mit 7 bis 20 Kohlenstoffatomen, ganz besonders bevorzugt von Cyclooctanon und Cyclododecanon, mit Wasserstoffperoxid und Ammoniak.

Die Reaktion verläuft bezüglich der Ammoximation von Cycloalkanonen hochgradig selektiv. Bei vollständigen Umsätzen liegt die Selektivität des Oxims nach gaschromatografischer Analyse (GC) von Cyclooctanon und Cyclododecanon jeweils bei über 99 %. Wird technisch reines Cyclododecanon eingesetzt, lassen sich als Nebenprodukte im Gaschromatogramm lediglich Spuren von Cyclododecan und Cyclododecanol nachweisen, welche bereits im Cyclododecanon als Verunreinigung vorhanden waren. Als weiteres Nebenprodukt wurde in wenigen Fällen Laurinlactam in Konzentrationen < 0,1 % nachgewiesen.

Als Lösemittel dienen Verbindungen, die gegenüber Wasserstoffperoxid und Ammoniak stabil sind und sowohl für die Carbonylverbindung als auch für das gebildete Oxim eine ausreichende Löslichkeit aufweisen. Das Lösemittel kann, muss aber nicht mit Wasser mischbar sein. Vorzugsweise eignen sich als Lösemittel aliphatische, mit Wasser mischbare oder teilweise mischbare Alkohole, ausgewählt aus C₁-C₆-aliphatischen oder cycloaliphatischen Alkoholen, wie zum Beispiel Methanol, Ethanol, n-Propanol, Isobutanol, tert.-Butanol oder tert.-Amylalkohol. Besonders geeignet sind für die Reaktion mit Cyclododecanon Methanol, Ethanol und tert.-Butanol.

Wasserstoffperoxid wird vorzugsweise als wässrige Lösung in handelsüblichen Konzentrationen (30 bis 70 %ig, bevorzugt mindestens 35 %ig) eingesetzt. Ammoniak wird entweder als konzentrierte, wässrige Lösung (vorzugsweise ≥ 20 %) oder bevorzugt als Gas dem Reaktor zugeführt. Vorteile ergeben sich bei der gasförmigen Dosierung des Ammoniaks und bei hoch konzentrierten Peroxidlösungen aus der geringeren Menge an Wasser, die bei der Aufarbeitung der Reaktionsmischung vom Lösemittel abgetrennt werden muss.

Die Reaktionstemperatur bei der Ammoximation liegt zwischen 20 °C und 150 °C, bevorzugt zwischen 50 °C und 120 °C und besonders bevorzugt zwischen 60 °C und 100 °C. Der Reaktor wird dabei entweder bei Normaldruck, definiert als der Druck, der sich durch die Summe der Partialdrucke bei den jeweiligen Reaktionstemperaturen einstellt, oder bei Überdruck, vorzugsweise zwischen 1 und 10 bar, betrieben. Der Überdruck kann mit Ammoniak oder einem Inertgas wie Stickstoff eingestellt werden. Wird der Reaktor verschlossen, steigt der Druck durch Bildung von gasförmigen Zersetzungsprodukten (vor allem Stickstoff) in Nebenreaktionen während der Reaktion langsam an. Vorteilhaft ist es, den Reaktor isobar zu betreiben, indem gasförmige Zersetzungsprodukte über einen leichten Abgasstrom kontrolliert entweichen können und dabei mit entweichender Ammoniak über ein Regelventil nachgedrückt wird.
Im Abgasstrom enthaltenes Ammoniakgas kann durch Kondensation aufgefangen und in den Prozess zurückgeführt werden.

Bei der Ammoximationsreaktion können Carbonylverbindung und Wasserstoffperoxid jeweils diskontinuierlich oder kontinuierlich zudosiert werden. Da Zersetzungsreaktionen gemäß Gleichung (3) immer nebenbei auftreten, wird für einen vollständigen Umsatz der Carbonylverbindung ein Überschuss an Peroxidlösung benötigt, welcher durch geeignete Reaktionsführung und die erfindungsgemäßen Katalysatorsysteme minimiert werden kann. Bei den Versuchen hat es sich als vorteilhaft erwiesen, zu Beginn der Reaktion entweder die Carbonylverbindung vorzulegen oder sie in äquimolaren Mengen parallel zum Wasserstoffperoxid zu dosieren und den benötigten Überschuss an Peroxid nach beendeter Zugabe der Carbonylverbindung gemäß dem Verbrauch nachzudosieren.

### Beispiele

Die nachfolgenden Beispiele sollen die Erfindung näher erläutern, ohne sie auf diese einzuschränken. Bei allen Beispielen wurde frischer Katalysator (Titansilikalit TS1, Degussa-Hüls AG) eingesetzt. Eine zusätzliche Aktivierung des Katalysators vor der Reaktion erfolgte nicht. Der pulverförmige Katalysator wurde nach der Reaktion über einen Druckfilter abgetrennt und so zurückgewonnen.

Beispiele 1 - 4 erfolgten in einem temperierten 100 ml-Druckreaktor aus Glas mit Begasungsrührer bei 60 °C und tert.-Butanol als Lösemittel. Die Reaktion wurde jeweils nach einer Gesamtzeit von etwas über 5 Stunden abgebrochen. Der Cyclododecanon-Umsatz wurde durch GC, der Peroxidgehalt durch Redoxtitration mit Cersulfat bestimmt.

### Beispiel 1 (Vergleichsversuch):

1,0 g Titansilikalit TS1 (Degussa-Hüls AG), 9,1 g Cyclododecanon, 1,5 g Diglyme (Diethylenglykoldimethylether) als interner Standard für die Gaschromatographie und 38,9 g 95 gew.-%iges tert.-Butanol wurden vorgelegt. Der Reaktor wurde mit Ammoniakgas gespült und ein Ammoniaküberdruck von 1,0 bar eingestellt. Nach Erhitzen auf 60 °C wurden unter intensivem Rühren innerhalb von 185 Minuten 6,8 g 50,7 gew.-%ige Wasserstoffperoxidlösung zudosiert. Nach 2 Stunden Nachreaktion bei 60 °C wurde ein Wasserstoffperoxidumsatz von 81 % und ein Cyclododecanonumsatz von 70 % bestimmt. Als Produkt der Umsetzung von Cyclododecanon wurde durch Gaschromatografie ausschließlich Cyclododecanonoxim gefunden.

### Beispiel 2 (Vergleichsversuch):

Der Versuch wurde analog zu Beispiel 1 durchgeführt. Zusätzlich zum Titansilikalit-Katalysator (1,0 g TS1, Degussa-Hüls AG) wurden 1,0 g Kieselgel 60 (Merck) entsprechend DE 195 21 011 A1 vorgelegt. Es wurde ein Wasserstoffperoxidumsatz von 97 % und ein Cyclododecanonumsatz von 60 % bestimmt. Als Produkt der Umsetzung von Cyclododecanon wurde ausschließlich Cyclododecanonoxim gefunden.

### Beispiel 3:

Der Versuch wurde analog zu Beispiel 1 durchgeführt. Als Katalysator wurde eine Mischung aus 0,8 g des Titansilikalit-Pulverkatalysators (TS1, Degussa-Hüls AG) und 0,2 g Al₂O₃ Pural SB (Condea) eingesetzt. Es wurde ein Wasserstoffperoxidumsatz von 86 % und ein Cyclododecanonumsatz von 79 % bestimmt. Als Produkt der Umsetzung von Cyclododecanon wurde ausschließlich Cyclododecanonoxim gefunden.

### Beispiel 4:

Der Versuch wurde analog zu Beispiel 1 durchgeführt. Es wurden 1,0 g eines Katalysators eingesetzt, der durch Extrudieren des Titansilikalit-Pulverkatalysators (TS1, Degussa-Hüls AG) mit 20 Gew.-% Al₂O₃ Pural SB, Trocknen, Calcinieren bei 550 °C und Pulverisieren der Extrudate hergestellt wurde. Es wurde ein Wasserstoffperoxidumsatz von 97 % und ein Cyclododecanonumsatz von 84 % bestimmt. Als Produkt der Umsetzung von Cyclododecanon wurde ausschließlich Cydododecanonoxim gefunden.

Die Beispiele 5 bis 11 zeigen die vollständige Umsetzung von Cyclododecanon unter optimierten Reaktionsbedingungen bei 80 °C in Ethanol als Lösemittel. Die Versuche erfolgten in einem temperierten 1,6 1-Druckreaktor aus Glas mit Gaseinleitungsrührer (500 U/min) und Druckregler. Über einen Probennehmer wurden in regelmäßigen Abständen Proben aus der Reaktionmischung entnommen und analysiert. Der Umsatz an Cyclododecanon wurde durch Gaschromatografie, Wasserstoffperoxid durch Redoxtitration bestimmt.

### Beispiel 5 (Vergleichsbeispiel, V):

2,5 g Katalysator (TS1, Degussa-Hüls AG) wurden bei 40 °C in einer Lösung von 62,7 g (344 mmol) Cyclododecanon in 488 g Ethanol suspendiert. Der Reaktor wurde auf 80 °C erwärmt und auf 0,1 bar entspannt. Anschließend wurde Ammoniakgas bis auf einen Druck von 1,6 bar aufgedrückt. Dabei wurden 13 g (765 mmol) Ammoniak zugegeben. Dies entspricht 2,2 MolÄquivalenten bezogen auf Cyclododecanon. Während der Reaktion wurde der Druck über einen leichten Abgasstrom konstant gehalten. Mit entwichenes Ammoniakgas (ca. 2 g / 4 h) wurde nachdosiert. Innerhalb von 2 Stunden wurden 2,04 Äquivalente (702 mmol) Wasserstoffperoxid als wässrige Lösung (50,4 gew.-%ig) zudosiert. Nach beendeter Peroxidzugabe ließ man die Reaktionsmischung noch 120 Minuten nachreagieren.

Nach 240 Minuten betrug der Umsatz 99,80 %. Es wurden 1,96 Äquivalente H₂O₂ verbraucht. Dies entspricht einer Peroxidselektivität von 50,9 %. Weitere Ergebnisse sind in den Tabellen 1 und 2 zusammengestellt.

### Beispiel 6:

Der Versuch wurde analog zu Beispiel 5 durchgeführt. Es wurden 2,5 g Titansilikalit (TS1, Degussa-Hüls AG) und 1,25 g Aluminiumoxid (Aldrich, aktiviert, saurer, ~ 150 mesh, CAMAG 504-C-I. Oberfläche 155 m²/g) als Cokatalysator verwendet. Innerhalb von 2 Stunden wurden 1,99 Äquivalente H₂O₂ dosiert. Nach 240 Minuten betrug der Umsatz 99,77 %, Es wurden 1,91 Äquivalente H₂O₂ verbraucht. Dies entspricht einer Peroxidselektivität von 52,2 %. Weitere Ergebnisse sind in den Tabellen 1 und 2 zusammengestellt.

### Beispiel 7:

Der Versuch wurde analog zu Beispiel 5 durchgeführt. Es wurden 2,5 g Titansilikalit (TS1, Degussa-Hüls AG) und 2,5 g Aluminiumoxid (Aldrich, wie Beispiel 6) als Cokatalysator verwendet. Innerhalb von 2 Stunden wurden 1,88 Äquivalente H₂O₂ dosiert. Nach 240 Minuten betrug der Umsatz 99,76 %. Es wurden 1,76 Äquivalente H₂O₂ verbraucht. Dies entspricht einer Peroxidselektivität von 56,7 %. Weitere Ergebnisse sind in den Tabellen 1 und 2 zusammengestellt.

### Beispiel 8:

Der Versuch wurde analog zu Beispiel 5 durchgeführt. Es wurden 2,5 g Titansilikalit (TS1, Degussa-Hüls AG) und 5,0 g Aluminiumoxid (Aldrich, wie Beispiel 6) als Cokatalysator verwendet. Innerhalb von 2 Stunden wurden 2,04 Äquivalente H₂O₂ dosiert. Nach 240 Minuten betrug der Umsatz 99,85 %. Es wurden 1,96 Äquivalente H₂O₂ verbraucht. Dies entspricht einer Peroxidselektivität von 51,0 %. Weitere Ergebnisse sind in den Tabellen 1 und 2 zusammengestellt.

### Beispiel 9:

Der Versuch wurde analog zu Beispiel 5 durchgeführt. Es wurden 2,5 g Titansilikalit (TS1, Degussa-Hüls AG) und 2,5 g Montmorrillonit (Engelhard, aktiviert, sauer, BET-Oberfläche 300 m²/g) als Cokatalysator verwendet. Innerhalb von 2 Stunden wurden 2,04 Äquivalente H₂O₂ dosiert. Nach 240 Minuten betrug der Umsatz 98,73 %. Es wurden 1,99 Äquivalente H₂O₂ verbraucht. Dies entspricht einer Peroxidselektivität von 49,8 %. Weitere Ergebnisse sind in den Tabellen 1 und 2 zusammengestellt.

### Beispiel 10:

Der Versuch wurde analog zu Beispiel 5 durchgeführt. Es wurden 2,5 g Titansilikalit (TS1, Degussa-Hüls AG) und 2,5 g Nafion NR 50 (Fluka) als Cokatalysator verwendet. Innerhalb von 2 Stunden wurden 2,04 Äquivalente H₂O₂ dosiert. Nach 240 Minuten betrug der Umsatz 99,84 %. Es wurden 1,94 Äquivalente H₂O₂ verbraucht. Dies entspricht einer Peroxidselektivität von 51,5 %. Weitere Ergebnisse sind in den Tab. 1 und 2 zusammengestellt.

### Beispiel 11 (Vergleichsbeispiel. V):

Der Versuch wurde analog zu Beisp. 5 durchgeführt. Es wurden 2,5 g Titansilikalit (TS1, Degussa-Hüls AG) und 2,5 g amorphes Siliziumdioxid (Kieselgel, Merck) als Cokatalysator verwendet. Innerhalb von 2 Stunden wurden 2,04 Äquivalente H₂O₂ dosiert. Nach 4 Stunden betrug der Umsatz 99,79 %. Es wurden 1,98 Äquivalente H₂O₂ verbraucht. Dies entspricht einer Peroxidselektivität von 50,4 %. Weitere Ergebnisse sind in den Tab. 1 und 2 aufgeführt.

**Tabelle 1: Umsatz CDON über die Reaktionszeit**

| **Beispiel Nr.** | **60 Min** | **120 Min** |
|---|---|---|
| (Cokatalysator) | [%] | [%] |
| **5** (ohne) (V) | 49,27 | 83,33 |
| **6** (1,25 g Aluminiumoxid) | 55,58 | 95,27 |
| **7** (2,5 g Aluminiumoxid) | 58,38 | 92,50 |
| **8** (5,0 g Aluminiumoxid) | 60,80 | 93,35 |
| **9** (2,5 g Montmorrillonit) | 56,41 | 93,86 |
| **10** (2,5 g Nafion) | 56,77 | 88,35 |
| **11** (2,5 g Kieselgel)(V) | 50,34 | 85,82 |

**Tabelle 2: Umsatzrate Oximbildung**

| **Beispiel Nr.** | **Umsatzrate nach 60 Min** |
|---|---|
| (Cokatalysator) | [mg Oxim/(g TS1•Min)] |
| **5** (ohne) (V) | 222,9 |
| **6** (1,25 g Aluminiumoxid) | 251,5 |
| **7** (2,5 g Aluminiumoxid) | 264,1 |
| **8** (5,0 g Aluminiumoxid) | 275,1 |
| **9** (2,5 g Montmorrillonit) | 255,2 |
| **10** (2,5 g Nafion) | 256,8 |
| **11** (2,5 g Kieselgel) (V) | 227,8 |

### Beispiele 12 (Vergleichsbeispiel):

Der Versuch wurde analog zu Beispiel 5 durchgeführt. Anstelle von Cyclododecanon wurden 43,67 g (346 mmol) Cyclooctanon vorgelegt. Innerhalb von 2 Stunden wurden 2,04 Äquivalente H₂O₂ dosiert, anschließend wurde 1 Stunde nachgerührt. Nach 60 Minuten waren 63,6 % Cyclooctanon zu Cyclooctanon-Oxim umgesetzt, dies entspricht einer Umsatzrate von 207,20 mg Oxim/(g TS1 · Min). Nach 180 Minuten betrug der Umsatz 99,7 %. 1,99 Äquivalente H₂O₂ wurden insgesamt verbraucht. Dies entspricht einer Peroxidselektivität von 50,1 %.

### Beispiel 13:

Der Versuch wurde analog zu Beispiel 12 durchgeführt. Es wurden zusätzlich 2,5 g Aluminiumoxid (Aldrich, wie Beispiel 6) als Cokatalysator eingesetzt. Nach 60 Minuten waren 71,9 % Cyclooctanon zu Cyclooctanon-Oxim umgesetzt, dies entspricht einer Umsatzrate von 234,3 mg Oxim/(g TS1 · Min) Nach 180 Minuten betrug der Umsatz 100 %. 2,01 Äquivalente H₂O₂ wurden insgesamt verbraucht. Dies entspricht einer Peroxidselektivität von 49,8 %.

### Beispiel 14:

Versuch 13 wurde wiederholt. Innerhalb von 2 Stunden wurden 1,51 Äquivalente H₂O₂ dosiert, anschließend wurde 1 Stunde nachgerührt. Nach 180 Minuten betrug der Umsatz 99,7 %. 1,50 Äquivalente H₂O₂ wurden verbraucht. Dies entspricht einer Peroxidselektivität von 66,5 %.

### Beispiel 15: Festbettreaktor

Der Versuchsaufbau aus Beispiel 5 wurde um einen Festbettreaktor mit Umwälzpumpe (150 ml/min) ergänzt. Als Festbettkatalysator wurden 30 g Formkörper (Granulat, 1 mm Durchmesser) verwendet, hergestellt im Extruder analog zu Beispiel 4 aus 50 Gew.-% Titansilikalit (TS1, Degussa-Hüls AG) und 50 Gew.-% Aluminiumoxid Pural SB.
Die Reaktion erfolgte bei 80 °C und konstantem Druck von 1,6 bar analog zu Beispiel 5. 62,7 g Cyclododecanon wurden in 488 g Ethanol vorgelegt. Wasserstoffperoxid (50 gew.-%ige wässrige Lösung) wurde vor dem Festbettreaktor in den Umlaufreaktor eingespeist. Innerhalb von 4 Stunden wurden 1,94 Äquivalente Wasserstoffperoxid zudosiert, nach beendeter Peroxidzugabe wurde noch 1 Stunde nachgerührt.
Der Umsatz an Cyclododecanon betrug nach 300 Minuten 99,7 % bei einem Peroxidverbrauch von 1,90 Äquivalenten Dies entspricht einer Peroxidselektivität von 52,5 %.

## Patentansprüche

1. Verfahren zur Herstellung von Oximen aus cyclischen Ketonen mit 7 bis 20 Kohlenstoffatomen, Wasserstoffperoxid und Ammoniak in Gegenwart eines Katalysatorsystems, das aus mindestens zwei Komponenten besteht,
**dadurch gekennzeichnet, dass**
- als Katalysator mindestens eine Komponente aus mindestens einem kristallinen mikro- oder mesoporösen Feststoff auf Basis von Titan, Silizium und Sauerstoff aufgebaut ist,
- als Cokatalysator mindestens eine weitere Komponente aus einem sauren Feststoff, der ein organisches oder anorganisches Trägermaterial enthält, besteht, wobei entweder das Trägermaterial selbst lewis- oder brönstedsaure Eigenschaften besitzt oder entsprechende lewis- oder brönstedsaure funktionelle Gruppen auf das Trägermaterial aufgebracht werden und die Auftragung solcher Gruppen physikalisch oder chemisch erfolgt
und
- dass keine zusätzliche Aktivierung des Katalysators vor der Reaktion erfolgt.

2. Verfahren gemäß Anspruch 1,
**dadurch gekennzeichnet, dass**
die Carbonylverbindung Cyclooctanon oder Cyclododecanon ist.

3. Verfahren gemäß einem der Ansprüche 1 oder 2,
**dadurch gekennzeichnet, dass**
ein organisches Lösemittel verwendet wird.

4. Verfahren gemäß Anspruch 3,
**dadurch gekennzeichnet, dass**
das organische Lösemittel ein mit Wasser mischbarer oder teilweise mischbarer Alkohol, ausgewählt aus der Gruppe der C₁-C₆-aliphatischen oder cycloaliphatischen Monoalkohole, ist.

5. Verfahren gemäß Anspruch 4,
**dadurch gekennzeichnet, dass**
der Alkohol Methanol, Ethanol oder tert.-Butanol ist.

6. Verfahren gemäß einem der Ansprüche 1 bis 5,
**dadurch gekennzeichnet, dass**
die Reaktionstemperatur 20 °C bis 150 °C beträgt.

7. Verfahren gemäß einem der Ansprüche 1 bis 6,
**dadurch gekennzeichnet, dass**
der Druck 1 bis 10 bar beträgt.

8. Verfahren gemäß einem der Ansprüche 1 bis 7,
**dadurch gekennzeichnet, dass**
Ammoniak gasförmig in den Reaktor eingespeist wird.

9. Verfahren gemäß einem der Ansprüche 1 bis 8,
**dadurch gekennzeichnet, dass**
Wasserstoffperoxid als mindestens 35 gew.-%ige Lösung eingesetzt wird.

10. Verfahren gemäß einem der Ansprüche 1 bis 9,
**dadurch gekennzeichnet, dass**
als Katalysator ein Titansilikalit verwendet wird.

11. Verfahren gemäß einem der Ansprüche 1 bis 10,
**dadurch gekennzeichnet, dass**
als Cokatalysator ein saurer, anorganischer Feststoff auf Basis von Aluminiumoxid oder Alumosilikat verwendet wird.

12. Verfahren gemäß einem der Ansprüche 1 bis 10,
**dadurch gekennzeichnet, dass**
als Cokatalysator ein organischer Feststoff auf Basis saurer oder stark saurer Ionenaustauscher verwendet wird.

13. Verfahren gemäß einem Ansprüche 1 bis 12,
**dadurch gekennzeichnet, dass**
das Gewichtsverhältnis Katalysator zu Cokatalysator 0,1 : 1 bis 10 : 1 beträgt.

14. Verfahren gemäß einem der Ansprüche 1 bis 13,
**dadurch gekennzeichnet, dass**
Katalysator und Cokatalysator in Pulverform eingesetzt werden.

15. Verfahren gemäß einem der Ansprüche 1 bis 13,
**dadurch gekennzeichnet, dass**
Katalysator und Cokatalysator als Formkörper eingesetzt werden.

16. Verfahren gemäß Anspruch 15,
**dadurch gekennzeichnet, dass**
ein Formkörper eingesetzt wird, bei dem der Cokatalysator gleichzeitig auch als Binder des Formkörpers fungiert.

## Claims

1. Process for preparing oximes from cyclic ketones having from 7 to 20 carbon atoms, hydrogen peroxide and ammonia in the presence of a catalyst system comprising at least two components,
**characterized in that**
- at least one component as catalyst comprises at least one crystalline microporous or mesoporous solid based on titanium, silicon and oxygen,
- at least one further component as cocatalyst consists of an acidic solid comprising an organic or inorganic support material, where either the support material itself has Lewis-acid or Brönsted-acid properties or appropriate Lewis-acid or Brönsted-acid functional groups are applied to the support material and the application of such groups is carried out either physically or chemically and
- no additional activation of the catalyst takes place prior to the reaction.

2. Process according to Claim 1, **characterized in that** the carbonyl compound is cyclooctanone or cyclododecanone.

3. Process according to Claim 1 or 2, **characterized in that** an organic solvent is used.

4. Process according to Claim 3, **characterized in that** the organic solvent is an alcohol which is miscible or partially miscible with water and is selected from the group consisting of C₁-C₆-aliphatic or cycloaliphatic monoalcohols.

5. Process according to Claim 4, **characterized in that** the alcohol is methanol, ethanol or tert-butanol.

6. Process according to any of Claims 1 to 5, **characterized in that** the reaction temperature is from 20°C to 150°C.

7. Process according to any of Claims 1 to 6, **characterized in that** the pressure is from 1 to 10 bar.

8. Process according to any of Claims 1 to 7, **characterized in that** the ammonia is fed in gaseous form into the reactor.

9. Process according to any of Claims 1 to 8, **characterized in that** the hydrogen peroxide is used as a solution having a concentration of at least 35% by weight.

10. Process according to any of Claims 1 to 9, **characterized in that** the catalyst used is a titanium silicalite.

11. Process according to any of Claims 1 to 10, **characterized in that** the cocatalyst used is an acidic, inorganic solid based on aluminum oxide or aluminosilicate.

12. Process according to any of Claims 1 to 10, **characterized in that** the cocatalyst used is an organic solid based on an acid or strong acid ion exchange resin.

13. Process according to any of Claims 1 to 12, **characterized in that** the weight ratio of catalyst to cocatalyst is from 0.1:1 to 10:1.

14. Process according to any of Claims 1 to 13, **characterized in that** catalyst and cocatalyst are used in powder form.

15. Process according to any of Claims 1 to 13, **characterized in that** catalyst and cocatalyst are used as shaped bodies.

16. Process according to Claim 15, **characterized in that** a shaped body in which the cocatalyst simultaneously functions as binder for the shaped body is used.

## Revendications

1. Procédé de préparation d'oximes à partir de cétones cycliques ayant 7 à 20 atomes de carbone, de peroxyde d'hydrogène et d'ammoniac en présence d'un système catalyseur qui est constitué d'au moins deux composants, **caractérisé en ce que**
- en tant que catalyseur, au moins un catalyseur est constitué d'au moins une matière solide cristalline microporeuse ou mésoporeuse à base de titane, de silicium et d'oxygène,
- en tant que cocatalyseur, au moins un autre composant est constitué d'une matière solide acide qui contient un matériau support organique ou inorganique, et soit le matériau support par lui-même possède les propriétés d'un acide de Lewis ou de Brönsted, soit des groupes à fonctionnalité acide de Lewis ou de Brönsted correspondants sont appliqués sur le matériau support, l'application de ces groupes ayant lieu par voie physique ou chimique, et
- aucune activation supplémentaire du catalyseur n'a lieu avant la réaction.

2. Procédé selon la revendication 1, **caractérisé en ce que** le composé carbonylé est la cyclooctanone ou la cyclododécanone.

3. Procédé selon l'une des revendications 1 ou 2, **caractérisé en ce qu'**on y utilise un solvant organique.

4. Procédé selon la revendication 3, **caractérisé en ce que** le solvant organique est un alcool miscible ou partiellement miscible à l'eau, choisi dans l'ensemble des monoalcools aliphatiques en C₁-C₆ ou cycloaliphatiques.

5. Procédé selon la revendication 4, **caractérisé en ce que** l'alcool est le méthanol, l'éthanol ou le tert-butanol.

6. Procédé selon l'une des revendications 1 à 5, **caractérisé en ce que** la température de réaction est de 20 à 150°C.

7. Procédé selon l'une des revendications 1 à 6, **caractérisé en ce que** la pression est de 1 à 10 bar.

8. Procédé selon l'une des revendications 1 à 7, **caractérisé en ce que** l'ammoniac est injecté dans le réacteur sous forme gazeuse.

9. Procédé selon l'une des revendications 1 à 8, **caractérisé en ce que** le peroxyde d'hydrogène est utilisé sous forme d'une solution à au moins 35 % en poids.

10. Procédé selon l'une des revendications 1 à 9, **caractérisé en ce qu'**on utilise en tant que catalyseur un silicalite au titane.

11. Procédé selon l'une des revendications 1 à 10, **caractérisé en ce qu'**on utilise en tant que cocatalyseur une matière solide inorganique acide à base d'oxyde d'aluminium ou d'un aluminosilicate.

12. Procédé selon l'une des revendications 1 à 10, **caractérisé en ce qu'**on utilise en tant que cocatalyseur une matière solide organique à base d'échangeurs ions acides ou fortement acides.

13. Procédé selon l'une des revendications 1 à 12, **caractérisé en ce que** le rapport en poids du catalyseur au cocatalyseur est de 0,1:1 à 10:1.

14. Procédé selon l'une des revendications 1 à 13, **caractérisé en ce que** le catalyseur et le cocatalyseur sont utilisés sous forme d'une poudre.

15. Procédé selon l'une des revendications 1 à 13, **caractérisé en ce que** le catalyseur et le cocatalyseur sont utilisés sous forme d'objets façonnés.

16. Procédé selon la revendication 15, **caractérisé en ce qu'**on utilise un objet façonné dans lequel le cocatalyseur joue simultanément aussi le rôle d'un liant pour l'objet façonné.
